# EUROPEAN PATENT SPECIFICATION

(11) **EP 2 648 796 B1**
(45) Date of publication and mention of the grant of the patent: **12.07.2017**
(21) Application number: 11846570.7
(22) Date of filing: 06.12.2011
(51) Int. Cl.: A61B 46/00, A61B 46/10

(54) **PORTAL FOR MEDICAL INSTRUMENTS**
PORT FÜR MEDIZINISCHE INSTRUMENTE
PORTIQUE POUR INSTRUMENTS MÉDICAUX

(30) Priority: 06.12.2010 US 420111 P
(43) Date of publication of application: 16.10.2013
(73) Proprietor: MAYO FOUNDATION FOR MEDICAL EDUCATION AND RESEARCH, Rochester, MN 55905 (US)
(72) Inventor: DYE, Kenneth R., Jacksonville, Florida 32224 (US); FEINGLASS, Neil G., Jacksonville, Florida 32225-5406 (US)
(74) Representative: Conroy, John
(86) International application number: PCT/US2011/063518
(87) International publication number: WO 2012/078620

(56) References cited:
- WO-A1-00/41614
- WO-A1-2009/037606
- US-A- 4 384 573
- US-A1- 2003 205 233
- US-A1- 2004 103 904
- US-A1- 2004 103 904
- US-A1- 2005 145 254
- US-A1- 2005 145 254
- US-A1- 2006 258 909
- US-A1- 2007 112 337
- US-B2- 7 275 544

## Description

### BACKGROUND

### 1. Technical Field

This document relates to portals that can be used to create a channel through a surgical drape without compromising the sterile operating field. For example, this document provides portal having an applicator frame and sheath that can attach to a surgical drape to create a surgical channel for non-sterile medical instruments to be introduced to the sterile surgical field.

### 2. Background Information

Surgical drapes are used to cover a patient during an operative procedure. They are generally constructed of a material that is impervious to blood and other bodily fluids. When preparing a patient for a surgery, the area of the incision (the surgical area or surgical field) is sterilized to help prevent infection. Sterilized surgical drapes are then typically placed about the sterilized surgical area. The surgical drapes provide a barrier that helps isolate the sterilized surgical area from non-sterilized surroundings.

Document WO 00/41614 A1 discloses a portal according to the preamble of appended claim 1.

### SUMMARY

The present invention provides methods and materials related to portals that can be used to create a channel through a surgical drape without compromising the sterile operating field, according to claims 1 and 6. Preferred embodiments are defined in appended claims 2- 5 and 7- 15. For example, a portal provided herein can include an applicator frame and sheath (e.g., flexible sheath) that is capable of creating a channel into the sterile operating field so that cutting edge technologies (e.g., cutting edge technologies that use probes and wands) can be used at the time of surgery, even when their use is unanticipated.

In general, operating rooms continue to apply advanced technology that is often blended with other medical modalities to better serve patients. Examples of such advanced technologies include, without limitation, surface echocardiography, transthoracic surface echocardiography (TTE), epiaortic scanning (EAS), vascular Doppler/flow probe imaging, and pacemaker/automatic internal cardiac defibrillator (AICD) interrogation and programming wands. The methods and materials provided herein can improve the clinical benefit of these devices by allowing them to be utilized directly on the surgical field even when their use is unanticipated prior to the start of the procedure.

In some cases, a medical instrument introduced into a surgical field can compromise sterility by unsheathed, non-sterile cords that are dragged across the sterile surgical field. Introducing medical instruments into the sterile operating field that are improperly sheathed can increase the patient's risk for infection. The methods and materials provided herein can allow surgeons to use medical technologies at the time of surgery and avoid compromising the sterility of the surgical field. In some cases, the methods and materials provided herein can be used in conjunction with, for example, a pacemaker wand or surface ultrasound device, to provide a surgeon immediate feedback before the patient's incisions have been closed, thereby reducing the possibility of re-exploration.

As described herein, a portal can include an applicator frame and a sheath. Such a portal can be mounted on the sterile side of a surgical drape via the applicator frame. The sheath can have a proximal and distal end. The proximal end can be attached to the applicator frame, and the distal end can longitudinally extend from the applicator frame to define a closed channel (e.g., a channel where the distal end is closed or lacks an opening) into the operative field. The sheath can include a spring such that the sheath can have a collapsed or retracted configuration when the portal is not in use. The spring can allow the sheath to extend longitudinally into the sterile field in a reversible manner, creating a channel closed at its distal end. The channel can encase the working end (e.g., a probe or wand) and cord of a non-sterile surgical instrument, such as ultrasound, echocardiography, and the like.

The portal can be attached to the drape via an applicator frame, which can be coated with an adhesive coating. In some cases, an adhesive coating can serve as the only means to attach the applicator frame to the surgical drape. The applicator frame comprises a backing. The backing can be attached to the surgical drape on the non-sterile side and can match the dimensions of the applicator frame such that the applicator frame and backing can couple together. In some cases, the backing can have an adhesive coating used to attach the backing to the surgical drape. In some cases, the applicator frame and backing can mechanically couple together, pinching the drape in between the mechanical connection. For example, the applicator frame can have protruding darts that couple with receiving channels on the backing. In some cases, the applicator frame can have a threaded portion, and the backing can be configured to receive the threaded portion.

This document also provides methods of using a portal to create a channel to allow the use of a non-sterile surgical instrument during a surgery in a manner that does not compromise the sterility of the sterile surgical field. The method of using a portal can include attaching the portal on the sterile side of a surgical drape. The portal can be attached via an applicator frame. The portal can include a sheath (e.g., a sterile sheath) that is connected to the applicator frame. The method can further include cutting an opening into the surgical drape where the applicator frame is attached, inserting a non-sterile surgical instrument through the opening and into the channel formed by the sheath, and advancing the non-sterile surgical instrument forward such that it remains enclosed by the sheath. The method can further include attaching a backing on the non-sterile side of the surgical drape that couples with the applicator frame. The non-sterile surgical instrument can be an echocardiography probe, a vascular Doppler/flow ultrasound imaging probe, or an automatic internal cardia defibrillator interrogation and programming wand. In some cases, the method can be performed by a clinician on the sterile side of the surgical drape in cooperation with a clinician on the non-sterile side of the surgical drape during a surgical procedure.

In addition, this document provides a medical kit for creating a channel that can be configured to allow the use of medical devices (e.g., sterile or non-sterile medical devices) in a sterile operating field without compromising the sterility of the operating field. The medical kit can include a portal having an applicator frame and a longitudinally extendable sheath. The portal provided in the medical kit can include a backing that is configured to couple with the applicator frame. In some cases, the portal can be packaged separately and can be used to create a channel (e.g., flexible channel) during a surgical operation. The applicator frame and backing of the portal can be configured with appropriate means to be attached to the surgical drape, such as adhesive coatings or mechanically couplings. In some cases, the portal can be pre-attached to a surgical drape. The surgical drape can have at least one visibly transmissive window. The portal can be attached within the visibly transmissive window.

In general, one aspect of this document features a portal comprising, or consisting essentially of, an applicator frame and a collapsible sheath capable of defining a channel when expanded, wherein the sheath comprises a proximal end and distal end, wherein the proximal end of the sheath is attached to the applicator frame, wherein the distal end of the sheath is closed such that the channel lacks a distal opening, wherein the applicator frame is configured to be attached to a sterile side of a surgical drape, and wherein the sheath is configured such that the distal end of the sheath is capable of extending away from the applicator frame to define the channel. The applicator frame can be coupled to a backing that is configured to be applied to a non-sterile side of the surgical drape. The applicator frame and the backing can comprise an adhesive coating. The applicator frame and the backing can be coupled via a mechanical connection. The applicator frame and the backing can be coupled via darts or threads. The sheath can comprise a spring configured to position the sheath in at least a partially collapsed position when the sheath is not in use. The channel can be configured to encase at least a portion of a surgical instrument introduced into the channel from a proximal end of the channel.

In another aspect, this document features a method for using a surgical instrument within a sterile surgical field. The method comprises, or consists essentially of, inserting at least a portion of the instrument into an opening at or near a proximal end of a channel defined by a sheath of a portal, wherein the portal comprising an applicator frame and the sheath, wherein the sheath comprises the proximal end and a distal end, wherein the proximal end of the sheath is attached to the applicator frame, wherein the distal end of the sheath is closed such that the channel lacks a distal opening, wherein the applicator frame is attached to a sterile side of a surgical drape defining the sterile surgical field, and wherein the distal end of the sheath is capable of extending away from the applicator frame to define the channel. The method can comprise attaching the applicator to the sterile side of the surgical drape. The attaching can comprise attaching a backing on a non-sterile side of the surgical drape that couples with the applicator frame. The method can comprise cutting an opening into the surgical drape at a position where the applicator frame is attached. The cutting can be performed by a technician on a non-sterile side of the surgical drape. The method can comprise advancing the sheath encased portion of the surgical instrument into the surgical field for use on a patient. The surgical instrument can be selected from the group consisting of echocardiography probes, vascular Doppler/flow ultrasound imaging probes, and automatic internal cardia defibrillator interrogation and programming wands. The method can be performed by a clinician on a sterile side of the surgical drape in cooperation with a clinician on a non-sterile side of the surgical drape during a surgical procedure.

In another aspect, this document features a medical kit comprising, or consisting essentially of, (a) an applicator frame configured to be attached to a surgical drape, wherein the applicator frame is attached to a proximal end of a sheath, wherein the sheath is configured such that a distal end of the sheath is capable of extending away from the applicator frame, and (b) a backing configured to couple with the applicator frame on a non-sterile side of the surgical drape. The applicator frame and backing can comprise an adhesive coating. The medical kit can comprise a surgical drape comprising at least one visibly transmissive window.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

Figures 1A, 1B, 1C, and 1D are side views of portals and surgical drapes according to some embodiments.
Figure 2A is a rear side angle view of an applicator frame of an exemplary portal.
Figure 2B is a rear side angle view of a backing that can couple with an applicator frame of an exemplary portal.
Figures 3A and 3B are side angle views of an applicator frame and backing of an exemplary portal that are coupled by darts.
Figures 4A and 4B are side angle views of an applicator frame and backing of an exemplary portal that are coupled by a threading mechanism.
Figures 5A and 5B is a front view of the sterile side of a surgical drape with an attached exemplary portal that has a cover panel.
Figure 6 is a front view of the non-sterile side of a surgical drape showing visible markings where an opening should be made.
Figures 7A and 7B are side views of an exemplary portal with an extended sheath that is attached to a surgical drape.
Figure 8 is a perspective view of the operation of an exemplary portal and surgical drape during a surgical procedure.
Figures 9A and 9B contains examples of processes for using an exemplary portal to create a channel that extends into the sterile field to encase a non-sterile device.
Figure 10 is a photograph of an exemplary wand instrument that can be introduced into a channel formed by a sheath of a portal provided herein.
Figure 11 is a front view (e.g., from the sterile side) of a surgical drape having a portal and a folding section according to some embodiments.
Figure 12 is a front view of the surgical drape of Figure 11 with the folding section partially folded to an open position.
Figure 13 is a front view of the surgical drape of Figure 11 with the folding section folded to an open position.
Figure 14 is rear view (e.g., from the non-sterile side) of a surgical drape having an inner drape channel with a portal according to some embodiments.
Figure 15 is a front view (e.g., from the sterile side) of a surgical drape having a portal and a pocket according to some embodiments.
Figure 16 is a side view of a surgical drape having a portal and first and second layers according to some embodiments.

### DETAILED DESCRIPTION

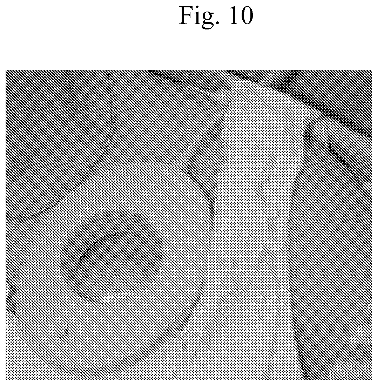

This document provides methods and materials related to portals that can be used to create a channel through a surgical drape without compromising a sterile operating field. A portal provided herein can include an applicator frame and a sheath (e.g., an integrated sheath) designed to attach to the sterile side of a surgical drape such that a non-sterile probe or wand (e.g., Figure 10) can be inserted through the portal and into an enclosed channel formed by the sheath, thereby maintaining the sterile nature of the surgical field. This device can allow a channel to be created during a surgical procedure when the need for such a probe or wand was unanticipated.

The term "sterile field" refers to an area or volume that can be created by placing a sterile surgical drape around a target surgical site to inhibit contamination. In this regard, the surgical drape may define an interface dividing a sterile field and a non-sterile field. Only sterile objects and personnel are typically allowed within a sterile field. Once a sterile object within a sterile field comes in contact with a non-sterile object, the "sterile" object is no longer sterile. The term "sterile" means that the component meets surgical cleanliness standards and is typically substantially aseptic and/or substantially free from living germs or microorganisms. The term "sterile side" refers to the side of the surgical drape facing the sterile surgical field. The term "non-sterile side" refers to the side of the surgical drape facing the non-sterile surgical field.

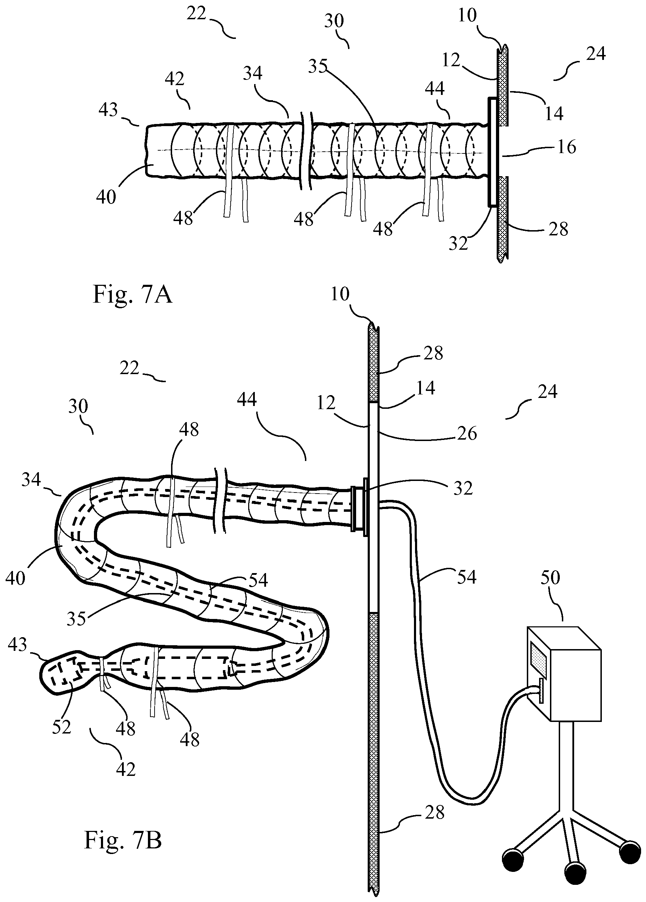

To assist in the description of the components of a portal provided herein, the following coordinate terms can be used. A "longitudinal axis" is generally parallel to a section of a sheath. In Figures 7A and 7B, the longitudinal axis is generally parallel to a closed channel 40 that is formed by an extended flexible sheath 34. As used herein, "the longitudinal direction" refers to a direction substantially parallel to the longitudinal axis. The term "proximal end" is used in reference to the portion of a sheath (e.g., sheath 34) near an applicator frame (e.g., applicator frame 32). The term "distal end" is used in reference to the end of a sheath (e.g., sheath 34) farthest from an applicator frame (e.g., applicator frame 32).

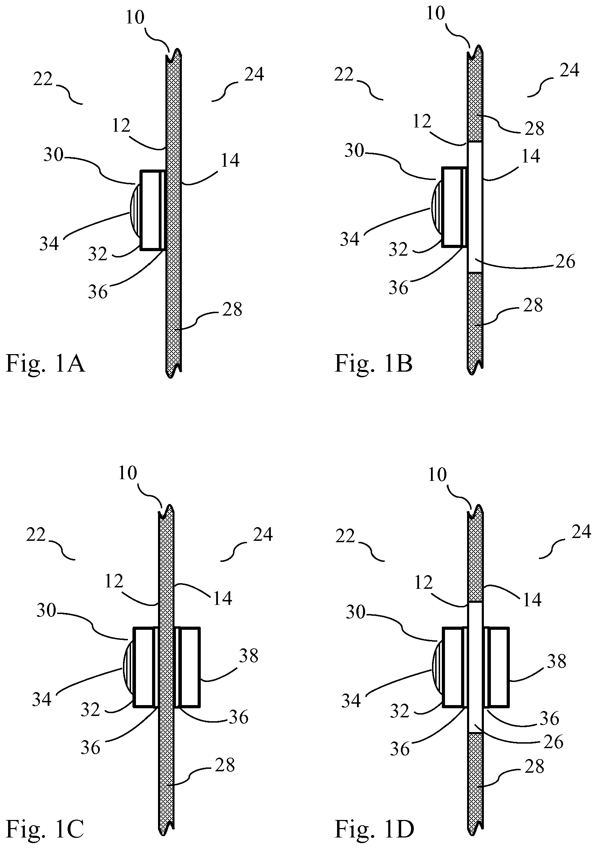

Figure 1A is a side view of a surgical drape 10. Surgical drape 10 can have opposing sides, a sterile side 12, and a non-sterile side 14. Sterile side 12 of surgical drape 10 can face sterile field 22, and non-sterile side 14 can face non-sterile field 24. Drape 10 is shown with a portal generally designated with reference number 30.

Portal 30 can be configured to be fixable and attached to sterile side 12 of surgical drape 10. Portal 30 can include an applicator frame 32 and a sheath 34 that is integrated with applicator frame 32. Sheath 34 can contain an elastic spring 35, which can be configured to allow the sheath to be retracted when not in use, as shown in Figures 1A-D. Figures 1C and 1D show an alternative embodiment of portal 30, where portal 30 includes a backing 36 that can couple with applicator frame 32 and is attached to non-sterile side 14 of surgical drape 10. Figures 1C and 1D are side views of a surgical drape 10 with an attached portal 30. Portal 30 shown in Figure 1C and 1D can include a backing 38 that couples with applicator frame 32 and is attached to surgical drape 10 on non-sterile side 14.

As shown in Figure 1A and 1C, portal 30 can be attached to a surgical drape 10 that is entirely made of opaque portion 28. Drapes used in practice can generally be entirely made out of opaque material commonly used for surgical drapes that meet the barrier performance requirements of AAMI PB70:2003 *Liquid Barrier Performance and Classification of Protective Apparel and Drapes Intended for Use in Health Car Facilities,* as established by the Association for the Advancement of Medical Instrumentation (Arlington, VA). The drape material can be gas sterilizable. Examples of opaque sheet material that can be used as described herein as a drape include, without limitation, woven or non-woven absorbant laminates, barrier composites, and barrier fabrics, such as those available from Precision Fabrics Group, Inc. (Vinton, VA). SMS non-woven material (e.g., spunbond-melt-blown-spunbond material) and/or 3M™ loban™ 2 antimicrobial drape material available from 3M (St. Paul, MN) can also be used in some applications. Although vertical drapes are shown throughout the document, it should be understood that a portal provided herein (e.g., portal 30) can be used in conjunction with any surgical drape (e.g., a horizontal drape, a vertical drape, or any other type of drape).

In some cases, surgical drape 10 can have at least one window 26 that is visually transmissive and an opaque portion 28 that may be translucent (e.g., not visually transmissive), as shown in Figures 1B and 1D. Surgical drape 10 can include two or more materials, one of which is visually transmissive and the other may be opaque. The visually transmissive window 26 of the surgical drape 10 can be constructed of an impervious polymer, such as polyethylene, polyvinylchloride, polypropylene, and combinations or mixtures thereof, for example. The visually transmissive window 26 can allow a clinician or anesthesiologist to view a patient and/or procedure there through. The transmissive window 14 can be secured to the opaque sheet material 28 with a seal that meets or exceeds the barrier performance of the drape material. For example, a suitable seal may be provided by heat fusion, ultrasonic welding, chemical adhesives, and/or combinations thereof, depending on the particular materials used for opaque portion 28 and visually transmissive window 26. The transmissive window 26 may be surrounded by opaque portions 28 or form an outer edge of surgical drape 10.

The transmissive window 26 is generally shown as rectangular or square, but may be of any convenient size and shape, including circular, oval, trapezoidal, and/or other polygonal or ovoid shapes, etc., suitable for attaching one portal 30 or more than one portal 30 and for clinician or anesthesiologist working in non-sterile field 24 to view the sterile field 22.

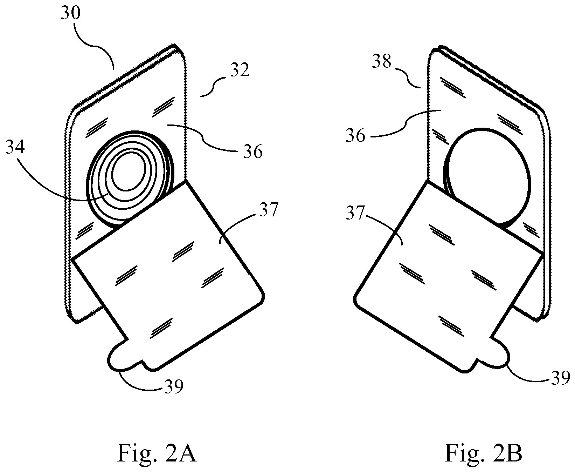

In some embodiments, such as when a portal provided herein (e.g., portal 30) is supplied separately from the surgical drape 10 (e.g., in a kit allowing a clinician to select the location of the portal 30 on the surgical drape 10, as described below), an adhesive layer 26 can be provided with a protective cover, such as a peel-away cover 37 (Figure 2A). Figure 2A illustrates a peel-away cover 37 half removed from applicator frame 32 revealing adhesive coating 36. Adhesive coating 36 can be used to secure applicator frame 32 to the sterile side 12 of surgical drape 10. Figure 2B is a similar view of backing 38 that can be coupled to applicator plate 32 on surgical drape 10. In this embodiment, backing 38 is an optional component. Backing 38 may provide additional support to portal 30, but is not required. Adhesive coating 36 can be used to attach backing 38 to non-sterile side 14 of surgical drape 10. The peel-away cover 37 can be configured to prevent adhesive layer 26 from inadvertently sticking to unintended objects and/or picking up any contaminants. Peel-away cover 37 can include a tab 39 that extends past the outer periphery of applicator frame 32. In some cases, tab 39 can be useful in removing peel-away cover 37 so as to expose adhesive coating 36 of applicator frame 32.

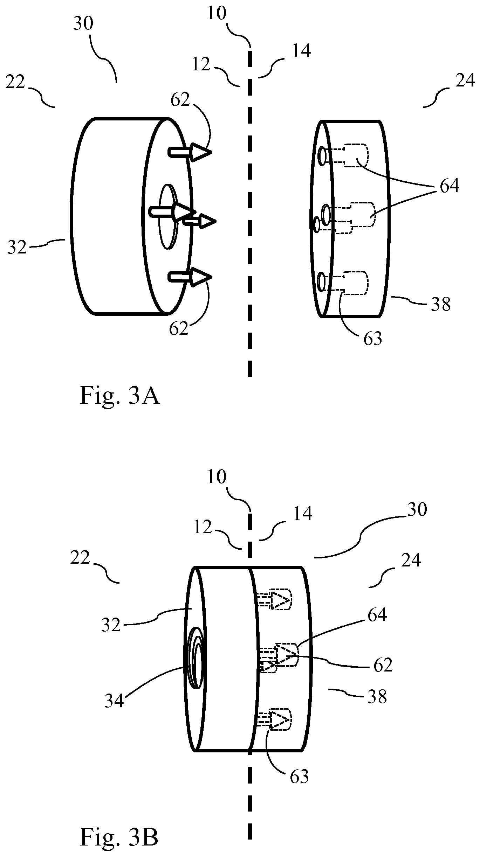

Figure 3 depicts examples of mechanical connections that can be used to connect portal 30 to surgical drape 10. In this figure, sterile drape 10 is represented by a dashed vertical line. Figure 3A shows applicator frame 32 with protruding darts 62. Although four darts 62 are shown on applicator frame 32, any number of darts could be used. For example, two, three, four, five, six, seven, eight, nine, ten, or more darts can be used. For this configuration, backing 38 can be used and configured accordingly with cavities 64 to receive darts 62 protruding from applicator frame 32. For each dart 62 on applicator frame 32, there can be a receiving cavity 64 in backing 38. Receiving cavities 64 can be configured to receive each dart 62 on applicator frame 32, pinching the surgical drape 10 in between dart 62 and receiving cavity 64. Once darts 62 have been pushed complete inside cavity 64, the widest part of darts 62 can catch on lip 63 in a manner such that they are difficult to remove. Figure 3B shows darts 62 pushed completely inside cavities 64 or backing 38 and retracted sheath 34 of portal 30. It should be understood that surgical drape 10 will be pinched between darts 62 and receiving cavities 64. Applicator frame 32 and backing 38 of Figure 3 may also have an adhesive coating 36 on the surfaces that attach to drape 10 to further secure the position of portal 30 on drape 10.

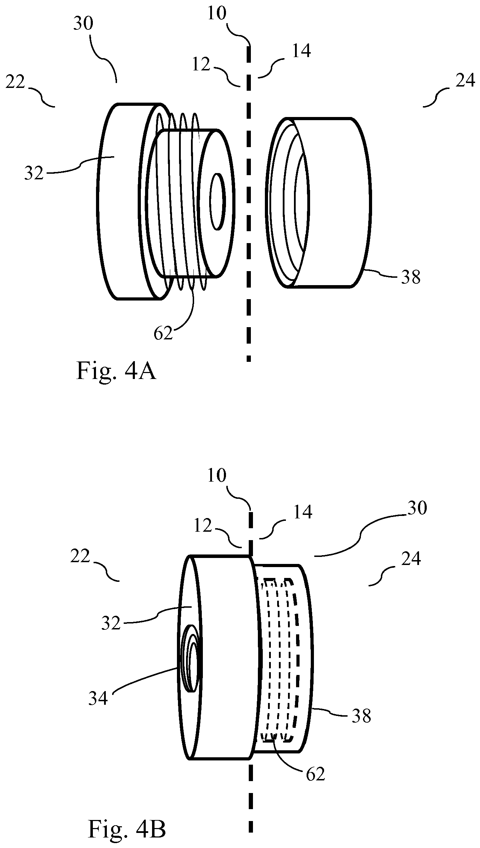

Referring now to Figure 4A, an applicator frame 32 depicts a screw-like connection configured to connect portal 30 on surgical drape 10, again represented by a vertical dashed line. Applicator frame 32 includes a threaded portion 66. For this configuration, a backing 38 can be used and configured accordingly to receive the threaded portion 66 of applicator frame 32. Backing 38 and applicator frame 32 can be connected by engaging the applicator frame 32 through the surgical drape and rotatably attaching the two pieces. The threads of the applicator frame 32 and backing 38 can be configured in such a way that they can be screwed together with surgical drape 10 between the threads of applicator frame 32 and backing 38. Figure 4B shows the applicator frame 32 and backing 38 screwed together. It should be understood that surgical drape 10 can be pinched between threads 66, securing the position of portal 30 on drape 10.

In some cases, an applicator frame and a backing can be configured to engage one another with a surgical drape located between the applicator frame and backing by way of magnetic force. For example, one or both of the applicator frame and backing can be magnetic such that the two can be held in proper position with a surgical drape located between the two.

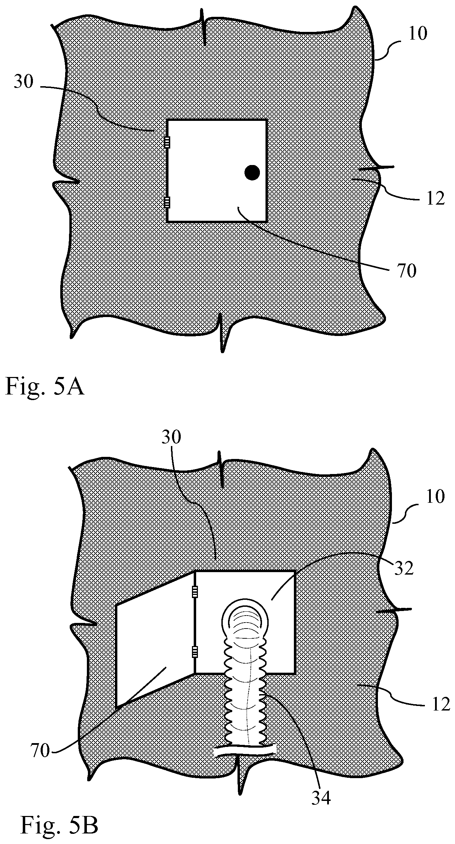

Figure 5A is a front view of the sterile side 12 of surgical drape 10. Shown attached to surgical drape is another embodiment of applicator frame 32. The applicator frame is shown with cover panel 70. The cover panel 70 can be a hinged door with a knob, as shown. The cover panel can also be a section of surgical drape that is affixed to applicator frame by an adhesive, Velcro, or any other reversible means of attachment. The cover panel 70 can remain closed when the portal 30 is not in use. When the clinician desires to use portal 30, the clinician can remove cover panel 70 so the sheath 34 can be extended. Figure 5B shows the cover panel 70 after it has been opened to reveal the sheath 34. A cover panel 70 is especially useful if the sheath 34 does not contain an internal spring 35 to keep it in a retracted configuration when not in use, as discussed below.

Applicator frame 32 and backing 38 can be made out of any suitable material, including molded plastic, silicone, polymer, fabric, textile, or a combination thereof and can provide increased structural rigidity to the surface of the surgical drape 10. The term "polymer" includes polymers, copolymers, and derivatives thereof.

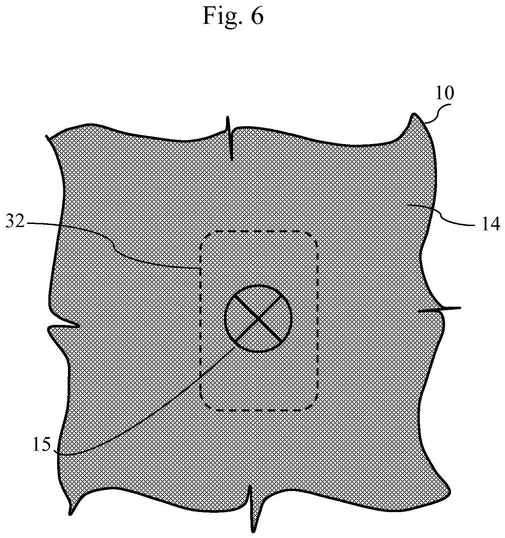

Portal 30 can be manufactured with the drape such that the portal is attached to surgical drape 10 in a pre-determined location. This may be preferred for common medical procedures, such as the use of ultrasound imaging for the placement of central lines. In some embodiments, portal 30 can be fixably attached to the sterile side 12 of surgical drape 10 before the drape 10 is provided (for example, during the manufacturing process). Figure 6 is a front view of a non-sterile side 12 of surgical drape 10. In some cases, surgical drape 10 can be made entirely of an opaque section 28. Applicator frame 32 of portal 30 can be attached on sterile side 12 of drape 10 as shown as a dotted outline. In order to create an opening 16 in the correct location to couple with portal 30, the drape 10 may have visible markings 15 on the non-sterile side 14 of drape 10. Visible markings 15 can show the clinician or anesthesiologist working in the non-sterile field 24 where an opening 16 should be made so that a non-sterile instrument can be introduced into sterile field 22 via an enclosed channel created by a sheath. The visible markings 15 can be useful when surgical drape 10 does not include a visibly transmissive window 26 and a backing 38 is not provided, as shown in Figure 6. Additional directions may be stamped, printed, or adhered to drape 10 to indicate how portal 30 should be attached and where opening 16 should be made. For example, explanatory text, arrows, and diagrams may be utilized and applied to surgical drape 10 in any of a wide variety of manners. The directions can be stamped on the sterile side 12, non-sterile side 14, or both sides of surgical drape 10.

In some cases, a portal provided herein (e.g., portal 30) can be manufactured separately from a surgical drape (e.g., surgical drape 10) and attached to a portion of the surgical drape during a surgical operation, or *in situ.* If a drape including at least one visually transmissive window (e.g., window 26) is supplied, the portal can be fixably attached to a portion of the surgical drape that is visually transmissive. In this regard, the visually transmissive window can also allow a clinician or anesthesiologist working in the non-sterile field to communicate with a clinician working in the sterile field to secure an applicator frame (e.g., applicator frame 32) and backing (e.g., backing 38). The visually transmissive window 26 can allow a clinician or anesthesiologist on the non-sterile side of a drape to view the instrument as it is being extended through drape 10 and portal 30 into the sterile field 22.

Figure 7A is a side view of the sheath 34 longitudinally extended as it would appear while in use in sterile field 22 and encasing a non-sterile instrument 50 (non-sterile instrument not shown for clarity). Although portal 30 is shown attached to surgical drape 10 made of one opaque section 28, it should be understood that the portal 30 could also be attached to a surgical drape 10 having one or more transmissive windows.

The sheath 34 can include a closure 48 to, for example, tighten sheath around working end 52 or cord 54 or non-sterile device 50. The closure 48 can be a drawstring, tie, ribbon, elastic band, or adhesive strip. For example, sheath 34 can have one or more than one closures 48 on the distal end portion 42. The sheath 34 can have none, one, or more than of closures 48 on the proximal end portion 44. If more than one closure 48 is provided on either the proximal or distal end portions, closures 48 can be the same type or different types. Referring to Figure 7A, sheath 34 is shown with three closures 48 that can be tied or tightened around an encased non-sterile instrument. In Figure 7B, sheath 34 is shown with a closure 48 on the distal end portion 42 that has been tied. Another closure 48 on distal end portion 42 of sheath 34 can remain untightened around working end 52 of device 50 (e.g., a non-sterile device 50). Another closure 48 on the proximal end portion 44 of sheath 34 is shown untied. The closures 48 can be loosened or tightened at any time during the surgical procedure by the clinician working in the sterile field 22. The closures 48 can be used to help control the sheath 34 or to reduce the volume of space taken up by the sheath 34.

Figure 7B is a side view of sheath 34 longitudinally extended and the encased working end 52 and cord 54 of non-sterile instrument 50. The working end 52 may be a probe or wand. The non-sterile instrument 50 may be, but is not limited to, a device designed to perform radiation monitoring, surface echocardiography, transthoracic surface echocardiography (TTE), epiaortic scanning (EAS), vascular Doppler/flow probe imaging or pacemaker/automatic internal cardiac defibrillator (AICD) interrogation and programming. In addition to the working end 52 of non-sterile instrument 50, sheath 52 can also enclose cord 54 that connects working end 52 to non-sterile instrument 50.

Working end 52 of non-sterile device 50 can be inserted into an opening 16 that has been cut into drape 10 from non-sterile side 14. Opening 16 can be created on the non-sterile side 14 of drape 10 by a clinician or anesthesiologist in the non-sterile field 24. If portal 30 is being created *in situ,* opening 16 can be made after the applicator frame 32 has been secured to the sterile side 12 of drape 10. In some cases, a cutting device can be included as part of the applicator frame of the portal.

As a device (e.g., a non-sterile device) is inserted into portal 30, the distal end 42 of sheath 34 can longitudinally extends away from proximal end portion 44 of sheath 34 that is connected to applicator frame 32. The longitudinally extended sheath 34 can creates a channel 40 that will encase a non-sterile device, thereby preventing it from contaminating the sterile surgical field 22. Flexible coils of spring 35 can allow distal end portion 42 of sheath 34 to retract towards proximal end 44 of sheath 34, such that the channel 40 can collapse when not in use. In some cases, sheath 34 can lack an internal spring 35. In such cases, sheath 34 can have pleats, for example, such that it can be stored in a collapsed configuration. With this type of sheath, applicator frame 32 can have a cover panel 70, as shown in Figures 5A and 5B. As depicted in Figure 5B, once cover panel 70 is opened, sheath 36 can unfold.

A sheath provided herein (e.g., sheath 34) can be fabricated from any suitable sterilizable, biocompatible polymeric material that is fluid-impermeable, such as latex, polyurethane, polyethylene, polycarbonate, or other suitable polymers. The sheath can function as a sterile barrier between the non-sterile device and the sterile field 22. Distal tip 43 can include coupling gel such as a pre-applied gel and cover as described in U.S. Patent No. 5,676,159. Distal tip 43 can include an ultrasound coupling interface as described in U.S. Patent Application Publication No. 2005/0215901.

With further reference to Figure 7B, extended sheath 34 can create a channel 40. The proximal end portion 44 can be open to receive working end 52 of non-sterile device 50. Distal end portion 42 of sheath 34 can be closed and define a form-fitting tip 43 that, in some cases, can conform in shape to working end 52 of non-sterile device 50. In some cases, form-fitting tip 43 can define a distal inner window extending generally transversely to the longitudinal axis of sheath. The form-fitting tip 43 can be clear, possessing no seams, fold lines, etc. and can have a planar outer surface so as to facilitate transmission and reception of any signals to or from the tissue.

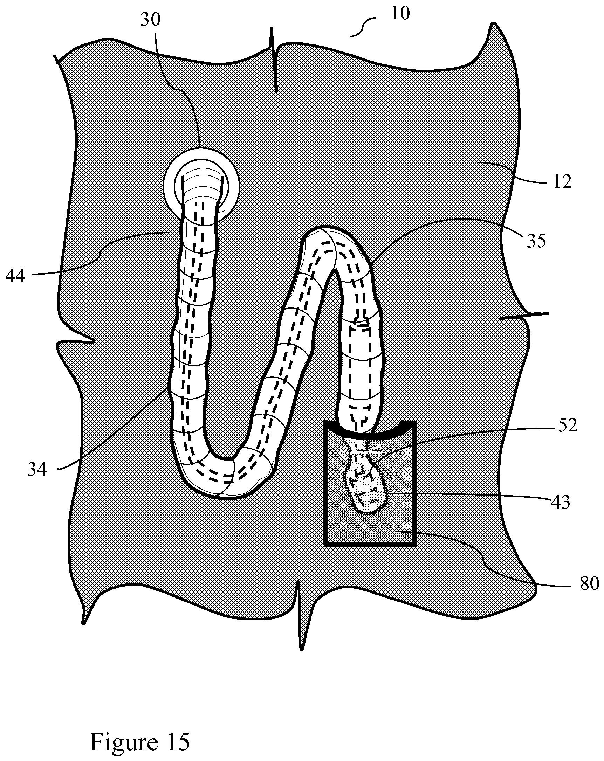

In some cases, as shown in Figure 15, a drape 10 can have a pocket or pouch 80 on a sterile side 12. Pocket 80 can be configured such that at least a distal tip 43 that contains a working end 52 of an instrument can be contained within pocket 80 when the instrument is not in use. In some cases, pocket 80 can be shaped such that nearly most or all of sheath 34 can be contained within a pocket when not in use. Although flexible coils 35 and ties 48 are shown in Figure 15, they are not required.

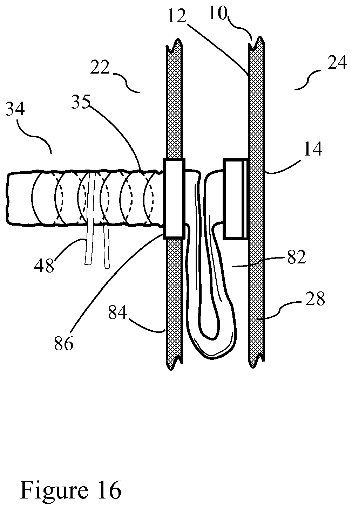

In another embodiment, with reference to Figure 16, a drape 10 can have a second layer 84 that can create a sterile space 82 between first layer 28 and second layer 84. Sterile space 82 can act as an internal pocket of drape 10. Second layer 84 can have an opening 86 for sheath 34 to pass through. Opening 86 can have a frame as described herein. Sterile space 82 can house all of sheath 34, a portion of sheath 34, or almost none of sheath 34, depending on the amount of sheath 34 required in sterile field 22. Although flexible coils 35 and ties 48 are shown in Figure 16, they are not required.

In some cases, a drape configured as shown in Figures 15 or 16 or similar to those shown in Figures 15 or 16 can include one or more visibly transmissive windows as described herein.

A sheath of a portal provided herein (e.g., sheath 34) can be configured to have an overall length in a fully extended condition that ranges from about 50 centimeters to about 5 meters (e.g., about 50 cm to about 4 m, about 50 cm to about 3 m, about 50 cm to about 2 m, about 90 cm to about 5 m, about 90 cm to about 4 m, about 90 cm to about 3 m, about 90 cm to about 2 m, about 1 m to about 3 m, or about 1.5 m to about 2 m). A sheath of a portal provided herein (e.g., sheath 34) can be configured to have an overall length in a completely compressed storage position that ranges from about 2 cm to about 20 cm (e.g., about 2 cm to about 18 cm, about 2 cm to about 15 cm, about 2 cm to about 10 cm, about 2 cm to about 5 cm, about 3 cm to about 20 cm, about 3 cm to about 18 cm, about 3 cm to about 15 cm, or about 5 cm to about 10 cm). The diameter of a proximal end portion of a sheath provided herein can range from about 4 cm to about 60 cm (e.g., about 4 cm to about 50 cm, about 4 cm to about 40 cm, about 4 cm to about 30 cm, about 5 cm to about 50 cm, about 5 cm to about 40 cm, or about 10 cm to about 30 cm), and the diameter of distal end portion can range from about 4 cm to about 40 cm (e.g., about 4 cm to about 30 cm, about 4 cm to about 20 cm, about 4 cm to about 15 cm, about 5 cm to about 30 cm, about 5 cm to about 20 cm, or about 10 cm to about 20 cm). In some cases, a sheath provided herein can have substantially similar diameter along substantially all of the sheath's length.

Sheath 34 can be secured and sealed to applicator frame 32 via conventional heat-sealing techniques. Any other technique, such as heat fusion, ultrasonic welding, chemical adhesion, and/or combinations thereof can be used to create a suitable seal.

It is noted that portal 30 in the embodiments shown in the figures are shown with a rectangular or circular applicator frame 32 and a sheath 34 with a circular diameter. Although portal 30 is shown with these particular shapes for applicator frame 32 and sheath 34, other shapes such as squares, ovals, triangles, hexagons, and other polygons or geometries may be used including irregular geometries. Portal 30 can provide an applicator frame 32 and sheath 34 that can accommodate a range of instrument sizes. For example, applicator frame 32 can be configured to accommodate instruments ranging between about 1 cm and about 15 cm in outer diameter. If backing 38 is supplied with portal 30, backing 38 can be of the appropriate dimensions to adequately couple with applicator frame 32.

In some cases, a surgical drape having a portal provided herein can be a folded surgical drape. For example, a surgical drape having a portal provided herein can be a surgical drape having one, two, three, four, or more folds. In some cases, a surgical drape having a portal provided herein can be a surgical drape having three folds with a first fold or seam extending through the portal, a second fold or seam extending to one side of the portal, and a third fold or seam extending to the other side of the portal. In such cases, unfolding of the surgical drape can expose a portal. Such an exposed portal can include a sheath and optionally include a frame and/or a protective cover (e.g., a peel-away cover 118 with a tab portion) as described herein. In some cases, adhesive layers or attachment portions can be used to hold the surgical drape in a folded position until it is ready for use. When ready for use, one or more of the folded sections held in a folded position by an adhesive or attachment portion can be detached or separated from other portions so as to unfold a section of the surgical drape in a manner that exposes a portal. Once exposed, the portal can be used as described herein.

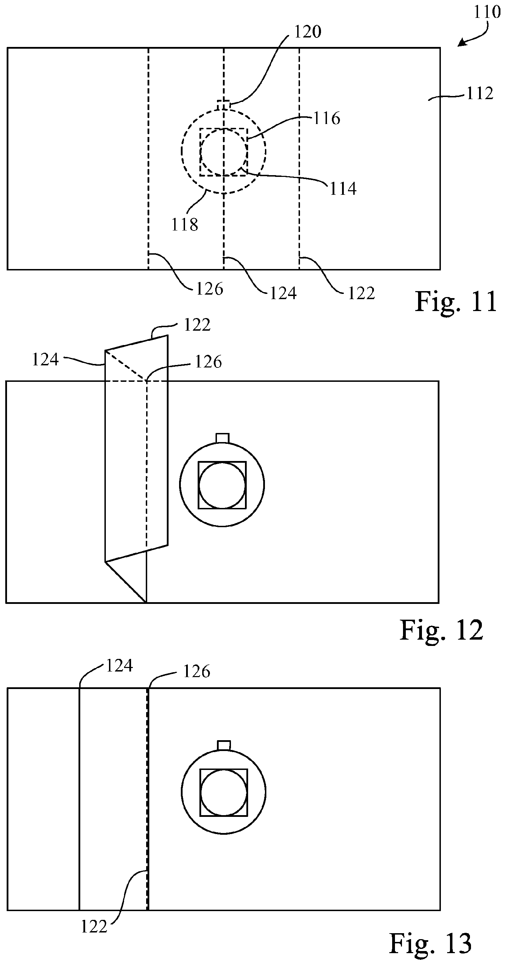

With reference to Figures 11-13, a surgical drape 110 can include a drape portion 112 and a folding section having predetermined folds 122, 124, and 126. Fold 122 can be configured to be extendable or removable from drape portion 112. For example, a folding section can swing away from drape section 112 once fold 122 is detached from drape portion 112 as shown in Figure 12. In such cases, a folding section can fold along folds 124 and 126 as shown. Further opening of a folding section can result in the folding section being positioned against or parallel with drape portion 112 as shown in Figure 13. In some cases, a folding section can be attached to drape section 112 in a manner that maintains the folding section in an open arrangement similar to that shown in Figure 13. For example, Velcro or an adhesive material can be used to maintain the folding section in an open arrangement as shown in Figure 13.

Once a folding section is extended away from drape section 112, a portal provided herein such as a portal 30 can be exposed to the sterile field. For example, as shown in Figures 11-13, a portal 114 and a frame 116 can be exposed. In some cases, portal 114 can include a protective cover, such as a peel-away cover 118 with a tab portion 120.

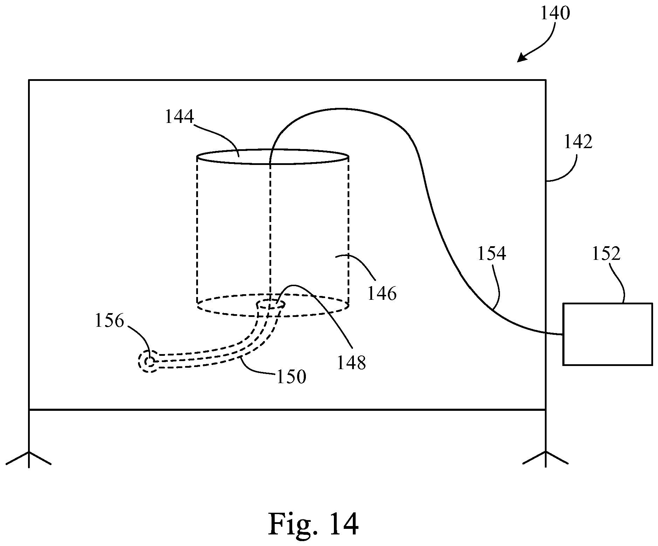

With reference to Figure 14, a surgical drape 140 can include a drape portion 142 and an inner channel section 146. In some cases, inner channel section 146 can be configured to be a pouch or channel formed between, for example, laminate layers of standard manufactured fiber based drape materials. Inner channel section 146 can define an opening 144 in drape portion 142 on the side of the non-sterile field and a closed end region on the side of the sterile field. The closed end region of inner channel section 146 can include a portal provided herein (e.g., portal 30). As shown in Figure 14, inner channel section 146 can include a portal 148 that includes a sheath 150. Sheath 150 creates a longitudinal channel that can allow the working end 156 and cord 154 of a device 152 to be used within a sterile surgical field without risking possible contamination of the sterile surgical field. The portal and/or sheath used in combination with drape portion 142 and inner channel section 146 can be configured as described herein (see, e.g., Figures 1-8).

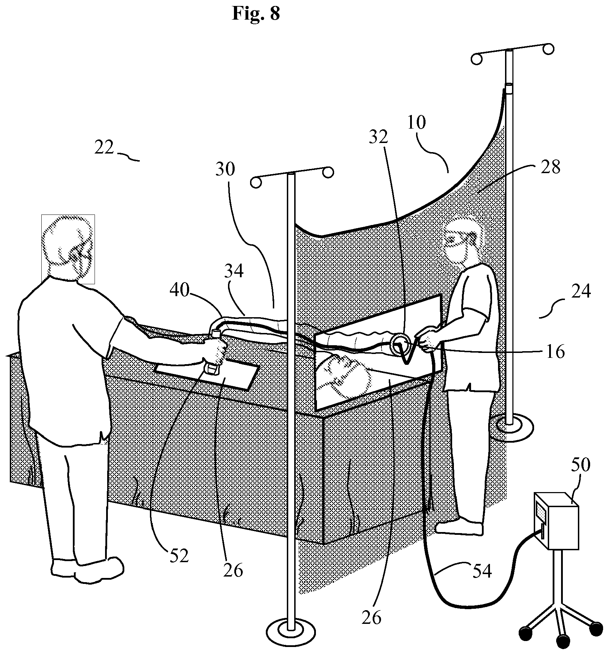

As described herein, one exemplary purpose of the portals provided herein (e.g., portal 30) is to allow instruments (e.g., instruments whether sterile or not) to be used in a sterile surgical field without contaminating the surgical field defined by a surgical drape (e.g., surgical drape 10). Figure 8 is a perspective view of an operating room with a portal 30 secured to surgical drape 10 via applicator frame 32. The extended sheath 34 creating a longitudinal channel 40 allows the working end and cord of device 50 to be used within sterile surgical field 22 without risking possible contamination of sterile surgical field 22.

Portal 30 including an applicator frame 32 and sheath 34 as described herein can be packaged as a medical kit. The medical kit can include at least one portal 30. The medical kit can include more than one, such as two, three, or a plurality of portals 30. Portals 30 can be all of the same size or different sizes, which can provide the same or different channel sizes. Portals 30 may be separately packaged or packaged together as a bundle in sterile packaging. One side of the portals 30 may have an adhesive coating that can be covered with a peel-away cover 37 as described herein. This configuration can be useful for some procedures or for some surgeons as it allows a clinician to arrange the surgical drape 10 and select the position of portal 30 as desired for a particular procedure *in situ.* Backings 38 may also be included in the kit. A backing 38 may be supplied for one or for all of the applicator frames 32. The backings 38 may be packaged separately from portals 30 since they can be positioned in the non-sterile field 24. The medical kit can optionally include the surgical drape 10. Optional surgical drape 10 can include at least one transparent window 26. In one embodiment, the medical kit can include a surgical drape 10 with a portal 30 pre-attached. Portal 30 can be affixed to drape 10 on an opaque section 28 or on a visibly transmissive window 26. In some embodiments, the medical kit can include a surgical drape 10 with more than one portal 30 pre-attached.

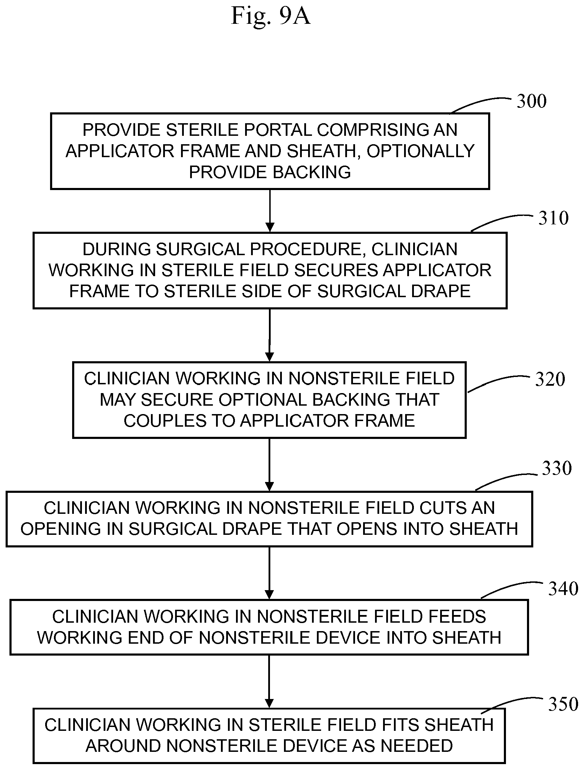

With reference to Figure 9A, a method of using a portal provided herein is shown and described. The following method is a method that can be used when portal 30 does not come pre-attached to surgical drape 10. At 300, a sterile portal is provided that includes an applicator frame and sheath. The sterile portal 30 provided can be packaged separately from the surgical drape 10 or packaged together with surgical drape 10, but is not pre-attached to surgical drape 10. At 310, if the use of a non-sterile device such as an ultrasound probe, echocardiograph wand, or similar device is desired during a surgical procedure, a clinician working in the sterile field 22 can secure the portal 30 via the applicator frame 32 to the sterile side 12 of surgical drape 10. The applicator frame 32 can be attached on a visually transmissive window 26 on the surgical drape 10. If surgical drape 10 does not have a visually transmissive window 26, the applicator frame 32 can be attached on any opaque portion 28 of the drape. At 320, a clinician working in the non-sterile field 24 can secure the backing 38, if provided, to the non-sterile side 14 of surgical drape 10. It should be understood that any of the embodiments described herein may be used to attach applicator frame 32 and backing 38 to surgical drape 10. In some cases, if the applicator frame 32 and backing 38 mechanically couple together, such as via the mechanisms shown in Figures 3A-4B, it should be understood that the clinicians working in the sterile field and non-sterile field may work cooperatively in order to couple the applicator frame 32 and backing 38. After portal 30 has been attached to drape 10 via the applicator frame 32, at 330, a clinician working in the non-sterile field 24 can cut an opening 16 through sterile drape 10 at the site. The opening 16 can be cut such that opening 16 results in access to the sheath 34 from the non-sterile field 24. At 340, a clinician working in the non-sterile field 24 can feed a working end 52 of a device 50 (whether sterile or not) into sheath 34. At 350, a clinician working the sterile field 22 can grab the sheath-encased working end 52 of device 50 and extend sheath 34 to create a longitudinal channel 40. If closures 48 are provided on sheath 34, the clinician can further adjust sheath 34 around working end 52 and cord 54 of device 50 as desired.

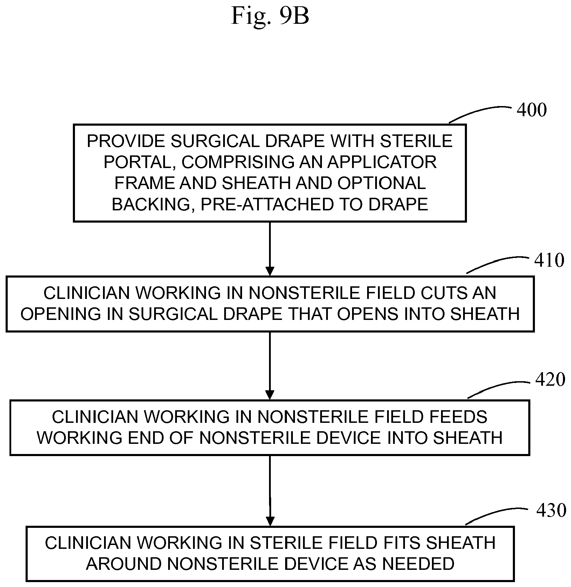

Figure 9B outlines the method that can be used in another embodiment. At 400, a portal 30 is provided pre-attached to surgical drape 10. The portal 30 can be pre-attached to surgical drape 10 on a visibly transmissive window 26 or on an opaque portion 28 of drape 10. In addition to applicator frame 32 and sheath 34, portal 30 may additionally have a backing 38. At 410, a clinician working in the non-sterile field 24 can cut on opening 16 through sterile drape 10 at the site. The opening 16 is cut such that opening 16 results in access to the sheath 34 from the non-sterile field 24. If portal 30 is attached on surgical drape 10 on a visibly transmissive window 26, the clinician in the non-sterile field 24 will be able to visualize where to make opening 16 since applicator frame 32 may be visible. If portal 30 is attached on the surgical drape 10 on an opaque section 28, the drape may have visible markings 15 which mark the area where opening 16 should be made. At 420, a clinician working in the non-sterile field 24 can feed working end 52 of a device 50 into sheath 34. At 430, a clinician working the sterile field 22 can grab the sheath-encased working end 52 of device 50 and extend sheath 34 to create a longitudinal channel 40. If closures 48 are provided on sheath 34, the clinician can further adjust the sheath 34 around the working end 52 and cord 54 of device 50 as desired.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A portal (30, 148) comprising
an applicator frame (32) and
a collapsible sheath (34) capable of defining a channel (40) when expanded,
wherein said sheath (34) comprises a proximal end (44) and distal end (42),
wherein said proximal end (44) of said sheath (34) is attached to said applicator frame (32),
wherein said distal end (42) of said sheath (34) is closed such that said channel (40) lacks a distal opening,
wherein said sheath (34) is configured such that said distal end (42) of said sheath (34) is capable of extending away from said applicator frame (32) to define said channel (40), **characterised in that** said applicator frame (32) is configured to be attached to a sterile side (12) of a surgical drape (10, 110, 140), and
wherein said applicator frame comprises a cover panel (70),
wherein said cover panel (70) is arranged to keep the sheath (34) in a retracted configuration when the portal (30, 148) is not in use.

2. The portal (30, 148) of claim 1, wherein the cover panel (70) is a section of surgical drape that is affixed to applicator frame by an adhesive, Velcro, or any other reversible means of attachment.

3. The portal (30, 148) of claim 1, wherein the cover panel (70) is configured to remain closed when the portal (30, 148) is not in use.

4. The portal (30, 148) of claim 1, wherein the cover panel (70) is a hinged door with a knob.

5. The portal (30, 148) of claim 1, wherein the surgical drape has a pocket or pouch (80) on the sterile side (12), wherein the pocket (80) is configured such that at least a distal tip (43) that contains a working end (52) of an instrument can be contained within said pocket (80) when the instrument is not in use.

6. A method for using a surgical instrument (50) within a sterile surgical field (22), wherein said method comprises
inserting at least a portion of said instrument (50) into an opening (16) at or near a proximal end (44) of a channel (40) defined by a sheath (34) of a portal (30, 148) according to claim 1.

7. The method of claim 6,
wherein said method comprises attaching said applicator to said sterile side (12) of said surgical drape (10, 110, 140),
in particular wherein said attaching comprises attaching a cover panel (70) on a sterile side (12) of said surgical drape (10, 110, 140) that couples with said applicator frame (32).

8. The method of claim 6, wherein said cover panel (70) is configured to be closed when the portal (30, 148) is not in use.

9. The method of claim 8, wherein closing the cover panel comprises affixing the cover panel (70), which is a section of surgical drape, to applicator frame by a knob, or an adhesive, or Velcro, or any other reversible means of attachment.

10. The method of claim 6,
wherein said method comprises cutting an opening (16) into said surgical drape (10, 110, 140) at a position where said applicator frame (32) is attached,
in particular wherein said cutting is performed by a technician on a non-sterile side (14) of said surgical drape (10, 110, 140).

11. The method of claim 6, wherein said method comprises advancing the sheath (34) encased portion of said surgical instrument (50) into said surgical field (22) for use on a patient.

12. The method of claim 6, wherein said surgical instrument (50) is selected from the group consisting of echocardiography probes, vascular Doppler/flow ultrasound imaging probes, and automatic internal cardia defibrillator interrogation and programming wands.

13. A medical kit comprising a portal (30, 148) according to claim 1.

14. The medical kit of claim 13,
wherein said cover panel is a hinged door with a knob, or wherein the cover panel (70) is a section of surgical drape that is affixed to applicator frame by an adhesive, Velcro, or any other reversible means of attachment.

15. The medical kit of claim 13, wherein said medical kit comprises a surgical drape (10, 110, 140) comprising at least one visibly transmissive window (14, 26).

## Patentansprüche

1. Port (30, 148), umfassend
einen Applikatorrahmen (32) und
eine zusammenklappbare Hülle (34), die einen Kanal (40) definieren kann, wenn sie expandiert ist,
wobei die Hülle (34) ein proximales Ende (44) und ein distales Ende (42) umfasst,
wobei das proximale Ende (44) der Hülle (34) an dem Applikatorrahmen (32) befestigt ist,
wobei das distale Ende (42) der Hülle (34) geschlossen ist, derart, dass der Kanal (40) keine distale Öffnung aufweist,
wobei die Hülle (34) derart ausgelegt ist, dass sich das distale Ende (42) der Hülle (34) vom Applikatorrahmen (32) weg erstrecken kann, um den Kanal (40) zu definieren,
**dadurch gekennzeichnet, dass**
der Applikatorrahmen (32) ausgelegt ist, an einer sterilen Seite (12) eines chirurgischen Abdecktuchs (10, 110, 140) befestigt zu werden, und
wobei der Applikatorrahmen eine Abdeckplatte (70) umfasst, wobei die Abdeckplatte (70) angeordnet ist, die Hülle (34) in einer zurückgezogenen Konfiguration zu halten, wenn der Port (30, 148) nicht benutzt wird.

2. Port (30, 148) nach Anspruch 1, wobei die Abdeckplatte (70) ein Abschnitt eines chirurgischen Abdecktuchs ist, das mit einem Klebstoff, Klettverschluss oder einem beliebigen anderen reversiblen Befestigungsmittel am Applikatorrahmen befestigt ist.

3. Port (30, 148) nach Anspruch 1, wobei die Abdeckplatte (70) ausgelegt ist, geschlossen zu bleiben, wenn der Port (30, 148) nicht benutzt wird.

4. Port (30, 148) nach Anspruch 1, wobei die Abdeckplatte (70) eine Klapptür mit einem Knopf ist.

5. Port (30, 148) nach Anspruch 1, wobei das chirurgische Abdecktuch einer Tasche oder einen Beutel (80) auf der sterilen Seite (12) aufweist, wobei die Tasche (80) derart ausgelegt ist, dass zumindest eine distale Spitze (43), die ein Arbeitsende (52) eines Instruments enthält, in der Tasche (80) enthalten sein kann, wenn das Instrument nicht benutzt wird.

6. Verfahren zur Verwendung eines chirurgischen Instruments (50) in einem sterilen Operationsfeld (22), wobei das Verfahren das Folgende umfasst:
Einführen zumindest eines Teils des Instruments (50) in eine Öffnung (16) an oder neben einem proximalen Ende (44) eines Kanals (40), der von einer Hülle (34) eines Ports (30, 148) nach Anspruch 1 definiert wird.

7. Verfahren nach Anspruch 6,
wobei das Verfahren das Befestigen des Applikators an
der sterilen Seite (12) des chirurgischen Abdecktuchs (10, 110, 140) umfasst,
wobei das Befestigen insbesondere das Befestigen einer Abdeckplatte (70) auf einer sterilen Seite (12) des chirurgischen Abdecktuchs (10, 110, 140) umfasst, die mit dem Applikatorrahmen (32) verbunden ist.

8. Verfahren nach Anspruch 6, wobei die Abdeckplatte (70) ausgelegt ist, geschlossen zu werden, wenn der Port (30, 148) nicht benutzt wird.

9. Verfahren nach Anspruch 8, wobei das Schließen
der Abdeckplatte das Befestigen der Abdeckplatte (70), die ein Abschnitt des chirurgischen Abdecktuchs ist, am Applikatorrahmen über einen Knopf oder einen Klebstoff oder Klettverschluss oder ein beliebiges anderes reversibles Befestigungsmittel umfasst.

10. Verfahren nach Anspruch 6,
wobei das Verfahren das Schneiden einer Öffnung (16) in das chirurgische Abdecktuch (10, 110, 140) an einer Stelle umfasst, an der der Applikatorrahmen (32) befestigt ist,
wobei das Schneiden insbesondere von einer medizinischtechnischen Fachkraft auf einer nichtsterilen Seite (14) des chirurgischen Abdecktuchs (10, 110, 140) durchgeführt wird.

11. Verfahren nach Anspruch 6, wobei das Verfahren das Vorschieben des von der Hülle (34) umschlossenen Abschnitts des chirurgischen Instruments (50) in das Operationsfeld (22) zur Verwendung an einem Patienten umfasst.

12. Verfahren nach Anspruch 6, wobei das chirurgische Instrument (50) aus der aus Echokardiographie-Sonden, Gefäß-Doppler/Durchfluss-Ultraschall-Bildgebungssonden und Abfrage- und Programmierstäben von implantierbaren Kardioverter-Defibrillatoren bestehenden Gruppe ausgewählt ist.

13. Medizinisches Kit, umfassend einen Port (30, 148) nach Anspruch 1.

14. Medizinisches Kit nach Anspruch 13,
wobei die Abdeckplatte eine Klapptür mit einem Knopf ist oder wobei die Abdeckplatte (70) ein Abschnitt eines chirurgischen Abdecktuchs ist, der mit einem Klebstoff, Klettverschluss oder einem beliebigen anderen reversiblen Befestigungsmittel am Applikatorrahmen befestigt ist.

15. Medizinisches Kit nach Anspruch 13, wobei das medizinische Kit ein chirurgisches Abdecktuch (10, 110, 140) umfasst, das mindestens ein sichtbar durchlässiges Fenster (14, 26) umfasst.

## Revendications

1. Portique (30, 148) comprenant:
un cadre d'applicateur (32); et
une gaine compressible (34) capable de définir un canal (40) lorsqu'elle est déployée,
dans lequel ladite gaine (34) présente une extrémité proximale (44) et une extrémité distale (42),
dans lequel ladite extrémité proximale (44) de ladite gaine (34) est attachée audit cadre d'applicateur (32),
dans lequel ladite extrémité distale (42) de ladite gaine (34) est fermée de telle sorte que ledit canal (40) ne comporte aucune ouverture distale,
dans lequel ladite gaine (34) est configurée de telle sorte que ladite extrémité distale (42) de ladite gaine (34) soit capable de s'étendre à l'écart dudit cadre d'applicateur (32) afin de définir ledit canal (40) ;
**caractérisé en ce que** ledit cadre d'applicateur (32) est configuré de manière à être attaché à un côté stérile (12) d'un drapage chirurgical (10, 110, 140), et
dans lequel ledit cadre d'applicateur comprend un panneau de recouvrement (70), dans lequel ledit panneau de recouvrement (70) est agencé de manière à maintenir la gaine (34) dans une configuration rétractée lorsque le portique (30, 148) n'est pas utilisé.

2. Portique (30, 148) selon la revendication 1, dans lequel le panneau de recouvrement (70) est une section de drapage chirurgical qui est fixée au cadre d'applicateur par un adhésif, une bande velcro ou tout autre moyen de fixation réversible.

3. Portique (30, 148) selon la revendication 1, dans lequel le panneau de recouvrement (70) est configuré de manière à rester fermé lorsque le portique (30, 148) n'est pas utilisé.

4. Portique (30, 148) selon la revendication 1, dans lequel le panneau de recouvrement (70) est une porte articulée munie d'un bouton.

5. Portique (30, 148) selon la revendication 1, dans lequel le drapage chirurgical comporte une poche ou une pochette (80) sur le côté stérile (12), dans lequel la poche (80) est configurée de telle sorte qu'au moins une pointe distale (43) qui contient une extrémité de travail (52) d'un instrument puisse être contenue à l'intérieur de ladite poche (80) lorsque l'instrument n'est pas utilisé.

6. Procédé d'utilisation d'un instrument chirurgical (50) à l'intérieur d'un champ chirurgical stérile (22), dans lequel ledit procédé comprend l'insertion d'au moins une partie dudit instrument (50) dans une ouverture (16) à ou à proximité d'une extrémité proximale (44) d'un canal (40) défini par une gaine (34) d'un portique (30, 148) selon la revendication 1.

7. Procédé selon la revendication 6, dans lequel ledit procédé comprend la fixation dudit applicateur audit côté stérile (12) dudit drapage chirurgical (10, 110, 140), en particulier dans lequel ladite fixation comprend la fixation d'un panneau de recouvrement (70) sur un côté stérile (12) dudit drapage chirurgical (10, 110, 140) qui se couple audit cadre d'applicateur (32).

8. Procédé selon la revendication 6, dans lequel ledit panneau de recouvrement (70) est configuré de manière à être fermé lorsque le portique (30, 148) n'est pas utilisé.

9. Procédé selon la revendication 8, dans lequel la fermeture dudit panneau de recouvrement comprend la fixation du panneau de recouvrement (70), qui est une section du drapage chirurgical, à un cadre d'applicateur par un bouton, un adhésif ou une bande velcro ou tout autre moyen de fixation réversible.

10. Procédé selon la revendication 6, dans lequel ledit procédé comprend la découpe d'une ouverture (16) dans ledit drapage chirurgical (10, 110, 140) à une position dans laquelle ledit cadre d'applicateur (32) est attaché, en particulier dans lequel ladite découpe est pratiquée par un technicien sur un côté non stérile (14) dudit drapage chirurgical (10, 110, 140).

11. Procédé selon la revendication 6, dans lequel ledit procédé comprend l'avancement de la partie enveloppée de la gaine (34) dudit instrument chirurgical (50) dans ledit champ chirurgical (22) en vue d'une utilisation sur un patient.

12. Procédé selon la revendication 6, dans lequel ledit instrument chirurgical (50) est sélectionné dans le groupe comprenant des sondes d'échocardiographie, des sondes d'imagerie par flux d'ultrasons/Doppler vasculaires et des baguettes d'interrogation et de programmation de défibrillateur cardiaque interne automatique.

13. Kit médical comprenant un portique (30, 148) selon la revendication 1.

14. Kit médical selon la revendication 13, dans lequel ledit panneau de recouvrement est une porte articulée munie d'un bouton, ou dans lequel le panneau de recouvrement (70) est une section de drapage chirurgical qui est fixée au cadre d'applicateur par un adhésif, une bande velcro ou tout autre moyen de fixation réversible.

15. Kit médical selon la revendication 13, dans lequel ledit kit médical comprend un drapage chirurgical (10, 110, 140) comprenant au moins une fenêtre visiblement transmissive (14, 26).
